# EUROPÄISCHE PATENTSCHRIFT

(11) **EP 3 778 868 B1**
(45) Veröffentlichungstag und Bekanntmachung des Hinweises auf die Patenterteilung: **26.01.2022**
(21) Anmeldenummer: 19192089.1
(22) Anmeldetag: 16.08.2019
(51) Int. Cl.: C12N 5/00

(54) **ZELLKULTURMEDIUM ZUR KULTIVIERUNG VON ZELLEN, VERFAHREN ZUM KULTIVIEREN VON ZELLEN UND VERFAHREN ZUR EXPRESSION VON MINDESTENS EINEM REKOMBINANTEN PROTEIN IN EINER ZELLKULTUR**
CELL CULTURE MEDIUM FOR CULTIVATING CELLS, METHOD FOR CULTURING CELLS AND METHOD FOR EXPRESSING AT LEAST ONE RECOMBINANT PROTEIN IN A CELL CULTURE
MILIEU DE CULTURE CELLULAIRE PERMETTANT DE CULTIVER DES CELLULES, PROCÉDÉ DE CULTURE DES CELLULES ET PROCÉDÉ D'EXPRESSION D'AU MOINS UNE PROTÉINE RECOMBINANT DANS UNE CULTURE CELLULAIRE

(43) Veröffentlichungstag der Anmeldung: 17.02.2021
(73) Patentinhaber: UGA Biopharma GmbH, 16761 Hennigsdorf (DE)
(72) Erfinder: Kober, Lars, 16761 Henningsdorf (DE); Herrmann, Sarah, 16761 Henningsdorf (DE)
(74) Vertreter: Pfenning, Meinig & Partner mbB

(56) Entgegenhaltungen:
- WO-A1-93/00423
- WO-A1-2016/156476
- WO-A2-01/16294
- US-A1- 2012 021 510
- LE ZHU ET AL: "Comparing Soluble Ferric Pyrophosphate to Common Iron Salts and Chelates as Sources of Bioavailable Iron in a Caco-2 Cell Culture Model", JOURNAL OF AGRICULTURAL AND FOOD CHEMISTRY, Bd. 57, Nr. 11, 18. Mai 2009 (2009-05-18), Seiten 5014-5019, XP55065932, US ISSN: 0021-8561, DOI: 10.1021/jf900328t
- KEENAN J ET AL: "Replacement of transferrin by simple iron compounds for MDCK cells grown and subcultured in serum-free medium", IN VITRO CELLULAR & DEVELOPMENTAL BIOLOGY. ANIMAL, SPRINGER US, NEW YORK, Bd. 32, Nr. 8, 1. September 1996 (1996-09-01), Seiten 451-453, XP009186846, ISSN: 1071-2690, DOI: 10.1007/BF02723044
- TIAN TIAN ET AL: "Dissolution behaviour of ferric pyrophosphate and its mixtures with soluble pyrophosphates: Potential strategy for increasing iron bioavailability", FOOD CHEMISTRY, ELSEVIER LTD, NL, Bd. 208, 23. März 2016 (2016-03-23), Seiten 97-102, XP029527322, ISSN: 0308-8146, DOI: 10.1016/J.FOODCHEM.2016.03.078
- GUPTA AJAY ET AL: "Physicochemical characterization of ferric pyrophosphate citrate", BIOMETALS, KLUWER ACADEMIC PUBLISHERS, NL, Bd. 31, Nr. 6, 15. Oktober 2018 (2018-10-15), Seiten 1091-1099, XP036641143, ISSN: 0966-0844, DOI: 10.1007/S10534-018-0151-1 [gefunden am 2018-10-15]

## Beschreibung

Es wird ein Zellkulturmedium zur Kultivierung von Zellen bereitgestellt, das als Eisenquelle einen Eisen-Citrat-Diphosphat-Komplex enthält. Ferner wird ein Verfahren zum Kultivieren von Zellen bereitgestellt, in dem ein Vermehren oder Halten einer oder mehrerer Zelle(n) in dem erfindungsgemäßen Zellkulturmedium durchgeführt wird. Zudem wird ein Verfahren zur Expression von mindestens einem rekombinanten Protein in einer Zellkultur vorgestellt, bei dem der in dem erfindungsgemäßen Zellkulturmedium vermehrten oder gehaltenen Zelle eine Nukleinsäure eingeführt wurde, die eine Produktion von mindestens einem rekombinanten Protein bewirkt. Das erfindungsgemäße Zellkulturmedium hat den Vorteil, dass sich die darin enthaltene Eisenquelle sehr gut und schnell in einer wässrigen Lösung (z.B. einem Zellkulturmedium, Zellkultursupplement oder Wasser) löst, effizient in den Zellinnenraum der Zellen importiert werden kann, eine erhöhte Lebendzellzahl und einen erhöhten Produkttiter (auch Produktkonzentration genannt) bei der Produktion rekombinanter Proteine bewirkt und sehr kostengünstig ist.

Zellkulturmedien zur Kultivierung biologischer Zellen können eine Eisenquelle enthalten, d.h. eine Quelle von Eisenionen, die von den biologischen Zellen effizient importiert werden kann. Zur Kultivierung von Säugetierzellen ist das Vorhandensein einer Eisenquelle im Zellkulturmedium sogar essentiell. Der Import von Eisenionen in die biologischen Zellen kann erreicht werden, indem einem Zellkulturmedium, das ein Eisensalz (z.B. Eisen(III)nitrat, Eisen(II)suIfat und/oder Eisen(III)chlorid) enthält, beispielsweise das Protein Transferrin zugeben wird, da Transferrin Eisenionen bindet und es in einer für die biologischen Zellen zugänglichen (importierbaren) Weise im Zellkulturmedium anbietet.

Transferrin wird gewöhnlich aus Blutplasma gewonnen oder rekombinant hergestellt und ist auch in trockener Form kommerziell erhältlich. Nachteil an dem erhältlichen Transferrin ist, dass es teuer und damit für die Zellkultur in industriellem Maßstab unökonomisch ist. Ferner ist das erhältliche Transferrin bereits teilweise mit Eisenionen gesättigt, sodass sich in dem Zellkulturmedium die Eisenkonzentration nicht exakt definiert einstellen lässt. Es besteht daher ein Bedarf an Transferrin-freien Zellkulturmedien zur Kultivierung von biologischen Zellen. Bei solchen Zellkulturmedien müssen die Eisenionen den biologischen Zellen in einer anderen Art und Weise effektiv biologisch zugänglich (d.h. in den Innenraum der Zellen importierbar) angeboten werden.

Im Stand der Technik ist es hierbei bekannt, den Zellkulturmedien das Eisen in anderer Form effektiv zugänglich zu machen.

Die CA 2 756 247 C offenbart, dass in einem Serum-freien Medium ein Eisensalz bzw. ein Eisen-Komplex als Eisenquelle verwendet werden kann. Beispielhaft wird genannt, dass die Eisenquelle ausgewählt sein kann aus der Gruppe bestehend aus Eisen(III)phosphat, Eisen(III)pyrophosphat, Eisen(III)nitrat, Eisen(II)sulfat, Eisen(111)chlorid, Eisen(II)laktat, Eisen(III)citrat, Ammonium-Eisen(II)citrat, Eisen-Dextran und EDTA-Eisen-Natriumsalz. Viele der aufgeführten Eisenquellen sind jedoch nicht dazu geeignet, den biologischen Zellen das Eisen auf eine gut zugängliche Art und Weise zur Verfügung zu stellen, sodass der Import der wichtigen Eisenionen in die Zellen erschwert ist. Die Folge ist, dass die Zellen langsamer wachsen und bei der Expression rekombinanter Proteine geringe Produkttiter zeigen. Eisen(II)citrat ist von den genannten Eisenquellen die effektivste, hat jedoch den Nachteil, dass es sich sehr langsam löst, falls es in einem trockenen Zellkulturmedium vorliegt und dieses mit Wasser zur Kultivierung der Zellen vorbereitet wird. Der lange Lösezeitraum von Eisen(II)citrat stellt einen Zeitnachteil bei der industriellen Herstellung von Zellkulturmedien zur Kultivierung von Zellen dar, der die Verwendung von Eisen(II)citrat unökonomisch macht.

Die WO 2016/156476 A1 offenbart, dass ein Transferrin-freies Zellkulturmedium, welches den biologischen Zellen Eisen über einen Eisen-Cholin-Citrat-Komplex zugänglich macht. Es wird gelehrt, dass der Eisen-Chlolin-Citrat-Komplex gegenüber gewöhnlicherweise verwendeten Eisenquellen, wie z.B. Eisen(II)phosphat, Eisen(III)pyrophosphat und Eisen(III)citrat, vorteilhaft ist, da er zu signifikant erhöhten Produktitern bei der Zellkultivierung beiträgt. Die Verwendung des Eisen-Cholin-Citrat-Komplexes in einem Zellkulturmedium ist jedoch mit dem Nachteil verbunden, dass die Herstellungskosten des Zellkulturmediums durch diesen Eisenkomplex sehr hoch sind und das Zellkulturmedium damit vor allem beim Bedarf von sehr großen Zellkultur-Volumina unökonomisch wird.

Zhu, L. et al. (Journal of Agricultural and Food Chemistry, Bd. 57, Nr. 11, S. 5014-5019) offenbaren, dass mit Eisenpyrophosphat (SFP) behandelte Caco-2-Zellen eine starke Bildung von Ferritin bewirkt und eine zusätzliche Zugabe von Citrat keinen signifikanten Effekt auf die Bioverfügbarkeit von SFP hat.

Die WO 93/00423 A1 offenbart ein Additiv für ein Kulturmedium, das ein Eisenchelat eines löslichen Eisensalzes und ein Alkalimetall- oder Erdalkalimetallcitrat umfasst, wobei das Additiv eine geeignete Eisenquelle für serumfreie oder proteinfreie Kulturmedien ist.

Die US 2012/021510 A1 offenbart ein Verfahren zur Kultivierung einer Zellpopulation in einem serumfreien Zellkulturmedium, wobei ein serumfreies Zellkulturmedium verwendet wird, das ein rekombinantes Albumin und ein rekombinantes Transferrin enthält.

Die WO 01/16294 A2 offenbart Metallbindungsverbindungen, die Zellkulturmedien zugesetzt werden können, um Faktoren zu ersetzen, die für die Kultivierung der Zellen (z. B. Transferrin) erforderlich sind, die tierischen oder menschlichen Ursprungs sind.

Keenan, J. et al. (In vitro cellular & developmental biology, Bd. 32, Nr. 8, S. 451-453) offenbaren den Ersatz von Transferrin durch einfache Eisenverbindungen für MDCK-Zellen, die in serumfreiem Medium gezüchtet und subkultiviert werden.

Tian Tian et al. (Food chemistry, Bd. 208, S. 97-102) offenbaren eine Studie über die Löslichkeit von FePP als Funktion des pH-Wertes und des Überschusses an Pyrophosphat-Ionen.

Gupta, A. et al. (Biometals, Bd. 31, Nr. 6, S. 1091-1099) offenbaren die physikochemischen Charakteristiken eines Eisen-Citrat-Diphophat-Komplexes.

Es war daher die Aufgabe der vorliegenden Erfindung, ein Zellkulturmedium bereitzustellen, das frei von Transferrin (bzw. ganz Serum-frei) ist und eine Eisenquelle enthält, mit der auf schnellere und kostengünstigere (ökonomischere) Art und Weise eine Zellkultivierung mit hohen Produkttitern erfolgen kann. Ferner sollte ein entsprechendes Verfahren zur Kultivierung von Zellen und ein entsprechendes Verfahren zur Expression von mindestens einem rekombinanten Protein in einer Zellkultur bereitgestellt werden.

Die Aufgabe wird gelöst durch das Zellkulturmedium mit den Merkmalen der Ansprüche 1 und 2, das Verfahren zur Kultivierung von Zellen mit den Merkmalen von Anspruch 9, das Verfahren zur Expression von mindestens einem rekombinanten Protein in einer Zellkultur mit den Merkmalen von Anspruch 11 und der Verwendung mit den Merkmalen von Anspruch 12. Die abhängigen Ansprüche zeigen vorteilhafte Weiterbildungen auf.

Erfindungsgemäß wird ein Zellkulturmedium bereitgestellt, das dadurch gekennzeichnet ist, dass es einen Eisen-Citrat-Diphosphat-Komplex enthält und in ungelöster Form vorliegt, wobei das Zellkulturmedium den Eisen-Citrat-Diphosphat-Komplex in einer Menge von 0,16 Gew.-% bis 12 Gew.-% enthält.

Ferner wird erfindungsgemäß ein Zellkulturmedium bereitgestellt, das dadurch gekennzeichnet ist, dass es einen Eisen-Citrat-Diphosphat-Komplex enthält und in gelöster Form vorliegt, wobei das gelöste Zellkulturmedium den Eisen-Citrat-Diphosphat-Komplex in einer Menge enthält, dass die über den Eisen-Citrat-Diphosphat-Komplex eingestellte Eisenkonzentration des Zellkulturmediums im Bereich von 80 µM bis 5800 µM liegt, wobei der Eisen-Citrat-Diphosphat-Komplex ein Eisen-Citrat-Diphosphat-Natrium-Komplex mit der CAS Nr. 85338-24-5 ist.

Unter dem Begriff "Zellkulturmedium" wird insbesondere ein Nährsubstrat verstanden, das zum Züchten und Erhalten von biologischen Zellen (z.B. Mikroorganismen, pflanzlichen, menschlichen und/oder tierischen Zellen), optional auch Viren, geeignet ist. Dieses Verständnis des Begriffs "Zellkulturmedium" lehnt sich an den HS-Code 38210000 des sogenannten "Harmonized Commodity Description and Coding Systems" (kurz: HS) an, das von der "World Customs Organization" (kurz: WCO) definiert wurde.

Das Zellkulturmedium kann auf der Dulbecco's Modified Eagle's Medium/Ham's nutrient Mixture F-12 (DMEM/F12) basieren.

Das Zellkulturmedium kann Spurenelemente und Salze enthalten, bevorzugt der Elemente Calcium, Eisen, Kobalt, Kupfer, Kalium, Magnesium, Mangan, Molybdän, Natrium, Nickel, Phosphat, Selen, Silizium, Zink und/oder Zinn (besonders bevorzugt von allen dieser Elemente).

Ferner kann das Zellkulturmedium essentielle und nichtessentielle Aminosäuren enthalten, bevorzugt Glycin, L-Alanin, L-Arginin, L-Asparagin, L-Asparaginsäure, L-Cystein, L-Cystin, L-Glutaminsäure, L-Glutamin, L-Histidin, L-Isoleucin, L-Leucin, L-Lysin, L-Methionin, L-Phenylalanin, L-Prolin, L-Serin, L-Threonin, L-Tryptophan, L-Tyrosin und/oder L-Valin (besonders bevorzugt alle davon).

Das Zellkulturmedium kann mindestens eine Komponente ausgewählt aus der Gruppe bestehend aus Biotin, Cholin, Folinsäure, Glukose, Hepes-Puffer, Hypoxanthin, Linolsäure, Liponsäure, Myoinositol, Niacinamid, Pantothensäure, Putrescin, Pyridoxal, Pyridoxin, Riboflavin, Thiamin, Tymidin, Pyruvat und Vitamin B12 enthalten (bevorzugt alle dieser Komponenten). Natriumhydrogencarbonat und/oder L-Glutamin können je nach Anwendung in dem Zellkulturmedium enthalten sein bzw. diesem zugegeben werden. So enthalten zum Beispiel pulverförmige Zellkulturmedien meist kein Natriumhydrogencarbonat. Dieses wird häufig erst während der Herstellung von flüssigen Zellkulturmedien zugesetzt. Da L-Glutamin in wässriger Lösung spontan zerfällt werden flüssigen Zellkulturmedien häufig ohne L-Glutamin herstellt, um deren Haltbarkeit zu erhöhen. L-Glutamin wird in diesen Fall, häufig erst kurz vor der Verwendung, als Stocksolution zugesetzt.

Das erfindungsgemäße Zellkulturmedium hat den Vorteil, dass es ohne Transferrin aus Blutserum oder ohne rekombinantes Transferrin als Eisenlieferant auskommt und gegenüber im Stand der Technik vergleichbaren Zellkulturmedien auf schnellere und kostengünstigere (ökonomischere) Art und Weise eine Zellkultivierung mit hohen Produkttitern ermöglicht, da der im Zellkulturmedium enthaltene Eisen-Citrat-Diphosphat-Komplex sich sehr schnell und gut in wässrigen Lösungen (z.B. Medien, Supplementen oder Wasser) löst und kostengünstiger ist als die bekannte Verwendung eines Eisen-Cholin-Citrat-Komplexes im Zellkulturmedium.

In einer bevorzugten Ausgestaltungsform liegt das Zellkulturmedium in ungelöster Form in Form eines Pulvers oder Granulats vor. Der Vorteil hierbei ist, dass das Zellkulturmedium eine lange Haltbarkeitsdauer aufweist und die Transportkosten geringer sind als im Falle eines Zellkulturmediums in gelöster Form. Das Zellkulturmedium enthält den Eisen-Citrat-Diphosphat-Komplex bevorzugt in einer Menge von 0,22 Gew.-% bis 6 Gew.-%, besonders bevorzugt 0,3 Gew.-% bis 2 Gew.-%, ganz besonders bevorzugt 0,4 Gew.-% bis 1 Gew.-%, insbesondere 0,5 Gew.-% bis 0,7 Gew.-%.

Das Zellkulturmedium kann in gelöster Form in Form einer wässrigen Lösung vorliegen. Der Vorteil hierbei ist, dass die Zeit für die Zugabe von Wasser und das Lösen des Zellkulturmediums in Wasser entfällt, sodass sofort mit der Kultivierung von biologischen Zellen begonnen werden kann. Das gelöste Zellkulturmedium enthält den Eisen-Citrat-Diphosphat-Komplex bevorzugt in einer Menge, dass die über den Eisen-Citrat-Diphosphat-Komplex eingestellte Eisenkonzentration des Zellkulturmediums im Bereich von 100 µM bis 3000 µM, besonders bevorzugt 150 µM bis 1000 µM, ganz besonders bevorzugt 200 µM bis 5000 µM, insbesondere 250 µM bis 350 µM, liegt.

Der Eisen-Citrat-Diphosphat-Komplex kann ausgewählt sein aus der Gruppe bestehend aus Eisen-Citrat-Diphosphat-Natrium-Komplex, Eisen-Citrat-Diphosphat-Kalium-Komplex, Eisen-Citrat-Diphosphat-Ammonium-Komplex und Mischungen hiervon. Bei dem Zellkulturmedium, das in gelöster Form vorliegt, handelt es sich erfindungsgemäß bei dem Eisen-Citrat-Diphosphat-Komplex um einen Eisen-Citrat-Diphosphat-Natrium-Komplex mit der CAS Nr. 85338-24-5. Bei dem Zellkulturmedium, das in ungelöster Form vorliegt, handelt es sich bei dem Eisen-Citrat-Diphosphat-Komplex bevorzugt um einen Eisen-Citrat-Diphosphat-Natrium-Komplex, besonders bevorzugt um einen Eisen-Citrat-Diphosphat-Natrium-Komplex mit der CAS Nr. 85338-24-5 und/oder EC-Nr. 286-697-4. Vorteil des Eisen-Citrat-Diphosphat-Natrium-Komplexes gegenüber den anderen Komplexen ist, dass Natriumionen - im Gegensatz zu anderen Kationen wie beispielsweise Ammonium - auch in höherer Konzentration im Zellkulturmedium von vielen biologischen Zellen toleriert werden.

In einer bevorzugten Ausgestaltungsform ist das Zellkulturmedium frei von mindestens einem Serumbestandteil, bevorzugt frei von Transferrin und/oder Lactotransferin.

Das Zellkulturmedium kann einen Serumersatz enthalten, bevorzugt einen UltroserG-Serumersatz in der im August 2018 erhältlichen Zusammensetzung. Hierbei ist das Zellkulturmedium insbesondere frei von Transferrin und/oder Lactotransferin.

Ferner kann das Zellkulturmedium Wachstumfaktoren enthalten. Hierbei ist das Zellkulturmedium insbesondere frei von Transferrin und/oder Lactotransferin.

In einer bevorzugten Ausgestaltungsform ist das Zellkulturmedium frei von tierischen oder humanen Komponenten ist.

Das Zellkulturmedium kann proteinfrei sein.

Ferner kann das Zellkulturmedium hydrolysatfrei sein.

Zudem kann das Zellkulturmedium chemisch-definiert sein.

Das Zellkulturmedium kann dadurch gekennzeichnet sein, dass es mindestens eine, bevorzugt mehrere, Aminosäure(n) enthält, wobei die mindestens eine, bevorzugt mehrere, Aminosäure(n) bevorzugt ausgewählt ist/sind aus der Gruppe bestehend aus Alanin, Arginin, Asparagin, Asparaginsäure, Cystein, Cystine, Glutamin, Glutaminsäure, Glycin, Histidin, Isoleucin, Leucin, Lysin, Methionin, Phenylalanin, Prolin, Serin, Threonin, Tryptophan, Tyrosin, Valin und Kombinationen und/oder Salze hiervon, wobei das Zellkulturmedium insbesondere alle dieser Aminosäuren enthält.

Ferner kann das Zellkulturmedium dadurch gekennzeichnet sein, dass es mindestens eine, bevorzugt mehrere, Lipid-Vorstufe(n) enthält, wobei die mindestens eine, bevorzugt mehrere Lipid-Vorstufe(n) bevorzugt ausgewählt sind aus der Gruppe bestehend aus Cholinchlorid, Ethanolamine, Glycerin, Inositol, Linolensäure, Fettsäure, Phopholipid, Cholesterin-verwandte Verbindungen und Kombinationen und Salze hiervon.

Zudem kann das Zellkulturmedium dadurch gekennzeichnet sein, dass es mindestens eine, bevorzugt mehrere, Carbonsäure(n) mit mindestens sechs C-Atomen enthält, wobei die mindestens eine, bevorzugt mehreren, Carbonsäure(n) bevorzugt ausgewählt ist/sind aus der Gruppe bestehend aus Linolsäure, Linolensäure, Thioctsäure, Ölsäure, Palmitinsäure, Stearinsäure, Arachinsäure, Arachidonsäure, Laurinsäure, Behensäure, Decansäure, Dodecansäure, Hexansäure, Lignocerinsäure, Myristinsäure, Octansäure und Kombinationen und Salze hiervon, wobei das Zellkulturmedium insbesondere alle dieser Fettsäuren und/oder Salze hiervon enthält.

Es ist möglich, dass das Zellkulturmedium dadurch gekennzeichnet ist, dass es mindestens eine, bevorzugt mehrere, Carbonsäure(n) mit weniger als sechs C-Atomen enthält, wobei die mindestens Carbonsäure bevorzugt Buttersäure oder ein Salz der Buttersäure ist.

In einer vorteilhaften Ausgestaltungsform ist das Zellkulturmedium dadurch gekennzeichnet, dass es mindestens ein, bevorzugt mehrere, Nukleosid(e) enthält, wobei das mindestens eine, bevorzugt mehrere, Nukleosid(e) ausgewählt sind aus der Gruppe bestehend aus Adenosin, Guanosin, Cytidin, Uridin, Thymidin, Hypoxanthin und Kombinationen und Salze hiervon, wobei das Zellkulturmedium insbesondere alle dieser Nukleoside und/oder Salze hiervon enthält.

Ferner kann das Zellkulturmedium dadurch gekennzeichnet sein, dass es mindestens ein, bevorzugt mehrere, Kohlenhydrat(e), enthält, wobei das mindestens eine, bevorzugt mehrere, Kohlenhydrat(e) bevorzugt ausgewählt ist/sind aus der Gruppe bestehend aus Glucose, Galactose, Glucosamin, Fructose, Mannose, Ribose, Saccharose und Kombinationen hiervon.

Das Zellkulturmedium kann dadurch gekennzeichnet sein, dass es mindestens eine, optional mehrere, Puffersubstanz(en) enthält, wobei die mindestens eine, optional mehrere, Puffersubstanz(en) bevorzugt ausgewählt ist/sind aus der Gruppe bestehend aus ACES, HEPES, MES, MOPS, NaHCO₃, PIPES, Phosphat-Puffer, TRIS und Kombinationen und/oder Salze hiervon.

Ferner kann das Zellkulturmedium sich dadurch auszeichnen, dass es mindestens ein, bevorzugt mehrere Spurenelement(e) enthält, optional ferner einen Chelatbildner, bevorzugt EDTA, enthält, wobei das mindestens eine, bevorzugt mehrere, Spurenelement(e) bevorzugt ausgewählt ist/sind aus der Gruppe bestehend aus Calcium, Kobalt, Kupfer, Kalium, Magnesium, Mangan, Molybdän, Natrium, Nickel, Phosphat, Selen, Vanadium, Zink, Zinn und Kombinationen hiervon, wobei das Spurenelement optional in Salzform vorliegen kann und wobei das Zellkulturmedium insbesondere alle dieser Spurenelemente enthält.

Zudem kann das Zellkulturmedium dadurch gekennzeichnet sein, dass es mindestens ein, bevorzugt mehrere Vitamin(e) enthält, wobei das mindestens eine, bevorzugt mehrere, Vitamin(e) bevorzugt ausgewählt ist/sind aus der Gruppe bestehend aus Biotin, Cholin, Folinsäure, Myoinositol, Niacinamid (B3), Pantothensäure, Pyridoxal, Pyridoxin, Riboflavin, Thiamin, Vitamin B12 and/or und Kombinationen und/oder Salze hiervon enthält.

In einer bevorzugten Ausgestaltungsform enthält das Zellkulturmedium, besonders bevorzugt in ungelöster Form, kein Eisensalz.

Ferner kann das Zellkulturmedium sich dadurch auszeichnen, dass es, besonders bevorzugt in ungelöster Form, kein Eisencitrat, bevorzugt kein Citratsalz und/oder keine Citronensäure, enthält.

Zudem kann das Zellkulturmedium dadurch gekennzeichnet sein, dass es, besonders bevorzugt in ungelöster Form, kein Eisendiphosphat, bevorzugt kein Diphosphatsalz und/oder keine Diphosphorsäure, enthält.

Das kann dadurch gekennzeichnet sein, dass es ein Zellkulturmedium ausgewählt aus der Gruppe bestehend aus DMEM, DMEM/F12 Media, Ham's F-10 Media, Ham's F-12 Media, Medium 199, MEM, RPMI 1640 Medium, ISF-1, Octomed, Ames' Medium, BGJb Medium (optional in der Fitton-Jackson Modification), Click's Medium, CMRL-1066 Medium, Fischer's Medium, Glascow Minimum Essential Medium (GMEM), Iscove's Modified Dulbecco's Medium (IMDM), L-15 Medium (Leibovitz), McCoy's 5A Modified Medium, NCTC Medium, Swim's S-77 Medium, Waymouth Medium, William's Medium E und Kombinationen hiervon, optional auch Modifikationen hiervon, enthält, wobei das jeweilige Zellkulturmedium in der im August 2018 erhältlichen Zusammensetzung gemeint ist.

In einer bevorzugten Ausgestaltungsform ist das Zellkulturmedium dadurch gekennzeichnet, dass es ein Zellkulturmedium ausgewählt aus der Gruppe bestehend aus DMEM, DMEM/F12 Media, Ham's F-10 Media, Ham's F-12 Media, Medium 199, MEM, RPMI 1640 Medium und Kombinationen hiervon, optional auch Modifikationen hiervon, enthält, wobei das jeweilige Zellkulturmedium in der im August 2018 erhältlichen Zusammensetzung gemeint ist.

Erfindungsgemäß wird ferner ein Verfahren zum Kultivieren von Zellen bereitgestellt, das die folgenden Schritte umfasst:
a) Bereitstellen eines Zellkulturmediums in einer wässrigen Lösung, wobei das gelöste Zellkulturmedium einen Eisen-Citrat-Diphosphat-Komplex in einer Menge enthält, dass die über den Eisen-Citrat-Diphosphat-Komplex eingestellte Eisenkonzentration des Zellkulturmediums im Bereich von 80 µM bis 5800 µM liegt, wobei der Eisen-Citrat-Diphosphat-Komplex ein Eisen-Citrat-Diphosphat-Natrium-Komplex mit der CAS Nr. 85338-24-5 ist; und
b) Vermehren oder Halten mindestens einer Zelle in der wässrigen Lösung des Zellkulturmediums.

Die Zelle kann hierbei eine Zelle einer Primärzelllinie oder einer kontinuierliche Zelllinie sein und bevorzugt ausgewählt sein aus der Gruppe bestehend aus Säugetierzelle, Vogelzelle und Insektenzelle, wobei die Zelle besonders bevorzugt eine Säugetierzelle ist, besonders bevorzugt eine Säugetierzelle ausgewählt aus der Gruppe bestehend aus CHO, N50, SP2/0, Hybridoma, HEK293, PERC-6, BHK-21 und Vero-76, ganz besonders bevorzugt eine CHO-Zelle, insbesondere eine CHO-Zelle ausgewählt aus der Gruppe bestehend aus CHO-DG44, CHO-DUKX, CHO-S und CHO-K1.

Zudem wird erfindungsgemäß ein Verfahren zur Expression von mindestens einem rekombinanten Protein in einer Zellkultur bereitgestellt, wobei hierbei das erfindungsgemäße Verfahren zum Kultivieren von Zellen ferner ein Einführen einer Nukleinsäure in die mindestens eine Zelle umfasst, wobei die Nukleinsäure eine konstitutive oder induzierte Produktion von mindestens einem rekombinanten Protein bewirkt, bevorzugt zusätzlich eine Sezernierung des produzierten Proteins in die wässrige Lösung des Zellkulturmediums bewirkt, wobei das rekombinante Protein besonders bevorzugt ausgewählt ist aus der Gruppe bestehend aus therapeutisches Protein, Antikörper, Fusionsprotein, Enzym, Vakzin, Biosimilar und Kombinationen hiervon.

Es wird erfindungsgemäß die Verwendung eines Eisen-Citrat-Diphosphat-Natrium-Komplex mit der CAS Nr. 85338-24-5 in einem Zellkulturmedium vorgeschlagen, wobei das Zellkulturmedium in gelöster Form vorliegt und einen Eisen-Citrat-Diphosphat-Komplex in einer Menge enthält, dass die über den Eisen-Citrat-Diphosphat-Komplex eingestellte Eisenkonzentration des Zellkulturmediums im Bereich von 80 µM bis 5800 µM liegt. Bevorzugt ist die Verwendung eine Verwendung eines Eisen-Citrat-Diphosphat-Komplexes als Inhaltsstoff in einem Zellkulturmedium für die Kultivierung von mindestens einer Zelle in dem Zellkulturmedium und/oder als Additiv in einem Zellkulturmedium während einer Kultivierung von mindestens einer Zelle in dem Zellkulturmedium. Der Eisen-Citrat-Diphosphat-Komplex kann dergestalt verwendet werden, dass das Zellkulturmedium mindestens eine der oben genannten Eigenschaften aufweist. Beispielsweise kann der Eisen-Citrat-Diphosphat-Komplex in einer der oben genannten Konzentrationen in dem Zellkulturmedium verwendet werden. Ferner kann der Eisen-Citrat-Diphosphat-Komplexes als Inhaltsstoff und/oder Additiv in einem Zellkulturmedium für die Kultivierung mindestens einer der oben genannten Zellen verwendet werden.

Anhand der nachfolgenden Beispiele und Figuren soll der erfindungsgemäße Gegenstand näher erläutert werden, ohne diesen auf die hier dargestellten, spezifischen Ausführungsformen einschränken zu wollen.

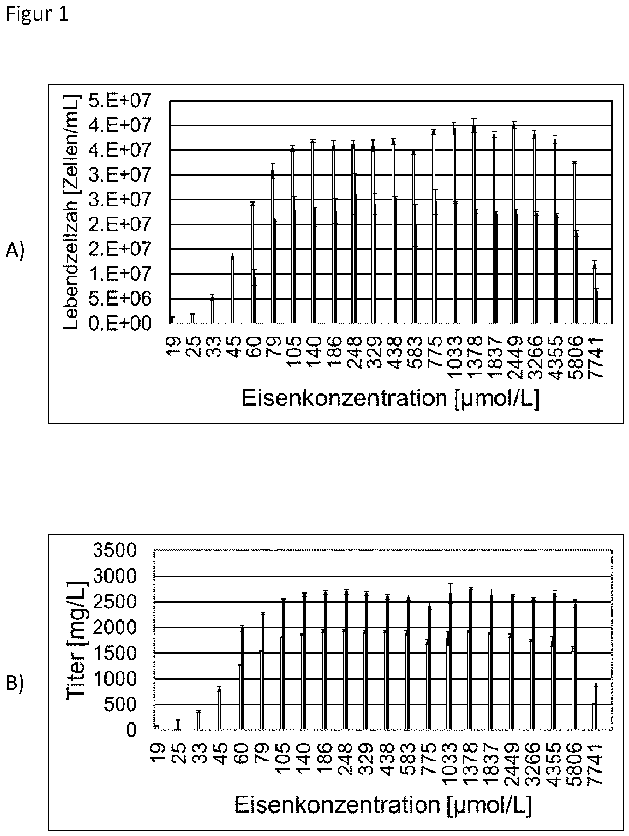

Figur 1 zeigt den Einfluss einer über einen Eisen-Citrat-Diphosphat-Natrium-Komplex eingestellten Eisenkonzentration (in µM) in einem Transferrin-freien flüssigen Kulturmedium auf das Zellwachstum und die Proteinproduktion von Zellen, die gentechnisch zur Herstellung eines bestimmten Proteins verändert wurden. Die x-Achse stellt die im flüssigen Medium eingestellte Eisenkonzentration dar. In Figur 1A sind auf der y-Achse die ermittelte Anzahl lebender Zellen (in Zellen pro mL) nach 10 Tagen (weiße Balken) und nach 13 Tagen (schwarze Balken) ab dem Beginn des Zellkultur-Experiments (Fed-Batch) dargestellt. In Figur 1B ist auf der y-Achse die ermittelte Produktkonzentration (Titer in mg/L) nach 10 Tagen (weiße Balken) und nach 13 Tagen (rechte schwarze Balken) ab dem Beginn des Zellkultur-Experiments (Fed-Batch) dargestellt. Es ist erkennbar, dass das Optimum der über den Eisen-Citrat-Diphosphat-Natrium-Komplex eingestellten Eisenkonzentration im Bereich von ca. 80 µM bis 5800 µM liegt.

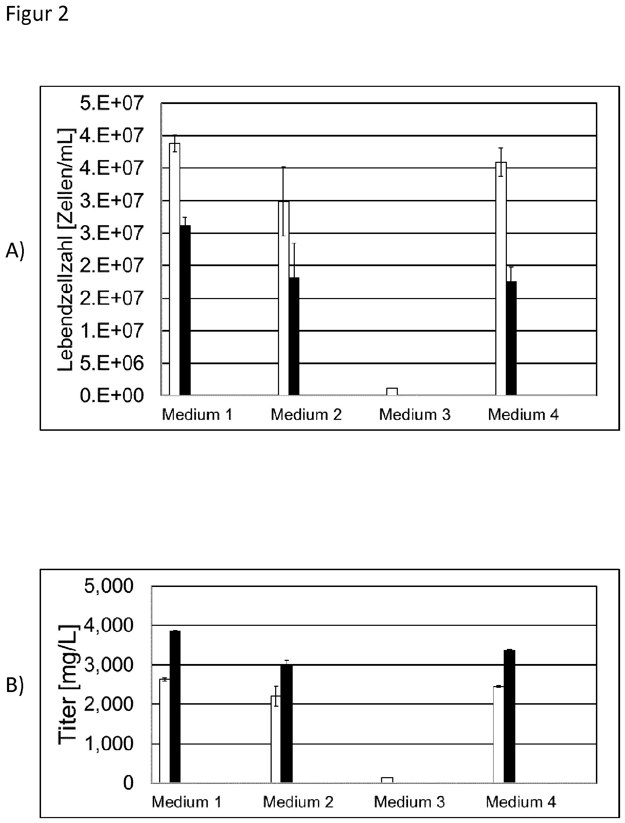

Figur 2 zeigt die Auswirkung einer über einen Eisen-Citrat-Diphosphat-Natrium-Komplex eingestellten Eisenkonzentration von 300 µM in einem Transferrin-freien flüssigen Kulturmedium auf das Zellwachstum und die Proteinproduktion von Zellen, die gentechnisch zur Herstellung eines bestimmten Proteins verändert wurden, im Vergleich zu einer über eine andere Eisenquelle eingestellten Eisenkonzentration von 300 µM in dem gleichen Medium. Anders ausgedrückt sind die vier auf der x-Achse dargestellten Medien 1, 2, 3, und 4 in ihrer Zusammensetzung identisch bis auf die eingesetzte Eisenquelle zur Einstellung der Eisenkonzentration von 300 µM. In Medium 1 ist die Eisenquelle ein Eisen-Citrat-Diphosphat-Natrium-Komplex, in Medium 2 ist die Eisenquelle Eisen(II)sulfat-Heptahydrat, in Medium 3 ist die Eisenquelle Eisen(III)nitrat-Nonahydrat, in Medium 4 ist die Eisenquelle Eisen(III)citrat. In Figur 2A sind auf der y-Achse die ermittelte Anzahl lebender Zellen (in Zellen pro mL) nach 10 Tagen (weiße Balken) und nach 13 Tagen (schwarze Balken) ab dem Beginn des Zellkultur-Experiments (Fed-Batch) dargestellt. In Figur 1B ist auf der y-Achse die ermittelte Produktkonzentration (Titer in mg/L) nach 10 Tagen (weiße Balken) und nach 13 Tagen (schwarze Balken) ab dem Beginn des Zellkultur-Experiments (Fed-Batch) dargestellt. Es ist erkennbar, dass die in Medium 1 enthaltene Eisenquelle (Eisen-Citrat-Diphosphat-Natrium-Komplex) gegenüber den in den Medien 2 bis 4 enthaltenen Eisenquellen nach 10 Tagen und nach 13 Tagen ab dem Beginn des Experiments eine vorteilhafte Wirkung auf die Anzahl der lebenden Zellen als auch auf die Produktkonzentration, d.h. auf die hergestellte Menge an Produkt, aufweist.

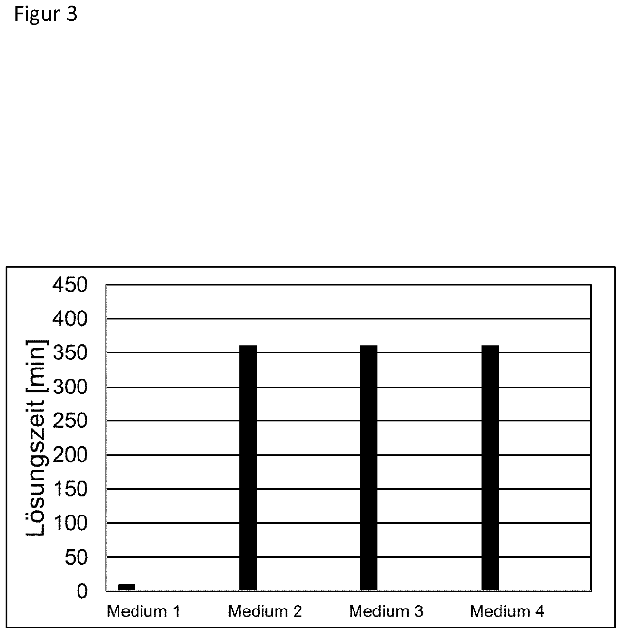

Figur 3 zeigt die Dauer bis zur vollständigen Lösung eines mit einem Eisen-Citrat-Diphosphat-Natrium-Komplex als Eisenquelle versetzten pulverförmigen Transferrin-freien Kulturmediums in Wasser im Vergleich zu Medien, welche die gleiche Zusammensetzung aufweisen und sich nur in der Eisenquelle unterscheiden. Anders ausgedrückt sind die vier auf der x-Achse dargestellten Medien 1, 2, 3 und 4 in ihrer Zusammensetzung identisch bis auf die eingesetzte Eisenquelle zur Einstellung einer Eisenkonzentration von 300 µM. In Medium 1 ist die Eisenquelle ein Eisen-Citrat-Diphosphat-Natrium-Komplex, in Medium 2 ist die Eisenquelle Eisen(II)sulfat-Heptahydrat, in Medium 3 ist die Eisenquelle Eisen(III)nitrat-Nonahydrat, in Medium 4 ist die Eisenquelle Eisen(III)citrat. Auf der y-Achse ist die Dauer (in Minuten) angegeben, bis sich das ursprünglich trockene, pulverförmige Medium vollständig in Wasser gelöst hat, wobei mehr als 360 Minuten benötigt wurden, um eine vollständige Lösung der ursprünglich trockene, pulverförmige Medien 2, 3 und 4 zu erreichen. Es ist erkennbar, dass sich das trockene, pulverförmige Medium 1 mit dem Eisen-Citrat-Diphosphat-Natrium-Komplex als Eisenquelle gegenüber den Medien 2, 3 und 4 die eine andere Eisenquelle aufweisen, deutlich schneller in Wasser löst.

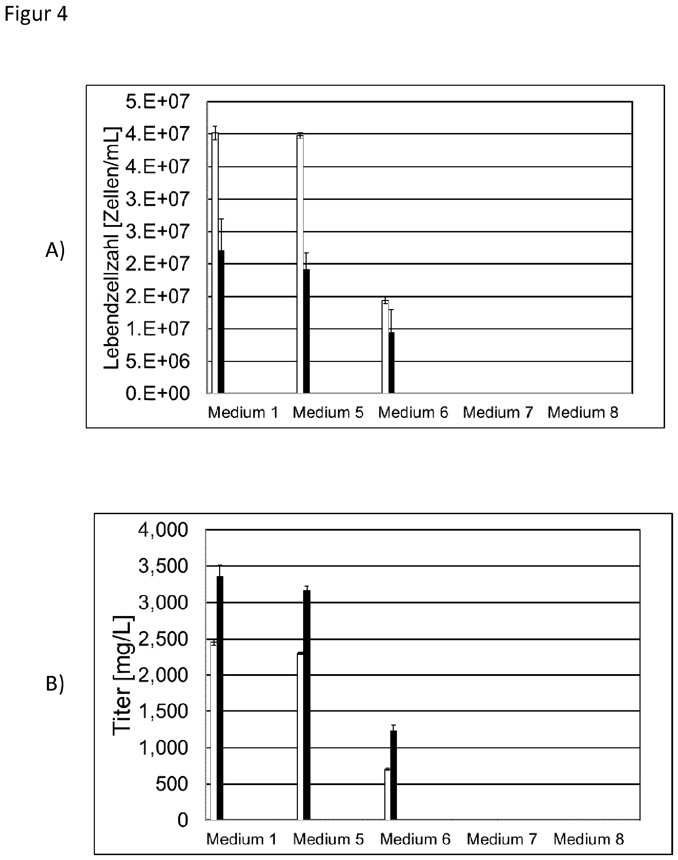

Figur 4 zeigt das Ergebnis eines weiteren Experiments zur Auswirkung einer über einen Eisen-Citrat-Diphosphat-Natrium-Komplex eingestellten Eisenkonzentration von 300 µM in einem Transferrin-freien flüssigen Kulturmedium auf das Zellwachstum und die Proteinproduktion von Zellen, die gentechnisch zur Herstellung eines bestimmten Proteins verändert wurden, im Vergleich zu einer über eine andere Eisenquelle eingestellten Eisenkonzentration von 300 µM in dem gleichen Medium. Anders ausgedrückt sind die fünf auf der x-Achse dargestellten Medien 1, 5, 6, 7 und 8 in ihrer Zusammensetzung identisch bis auf die eingesetzte Eisenquelle zur Einstellung der Eisenkonzentration von 300 µM. In Figur 4A sind auf der y-Achse die ermittelte Anzahl lebender Zellen (in Zellen pro mL) nach 10 Tagen (weiße Balken) und nach 13 Tagen (schwarze Balken) ab dem Beginn des Zellkultur-Experiments.(Fed-Batch) dargestellt. In Figur 1B ist auf der y-Achse die ermittelte Produktkonzentration (Titer in mg/L) nach 10 Tagen (weiße Balken) und nach 13 Tagen (schwarze Balken) ab dem Beginn des Zellkultur-Experiments (Fed-Batch) dargestellt. Es ist erkennbar, dass die in Medium 1 enthaltene Eisenquelle (Eisen-Citrat-Diphosphat-Natrium-Komplex) gegenüber den in den Medien 6 bis 8 enthaltenen Eisenquellen nach 10 Tagen und nach 13 Tagen ab dem Beginn des Experiments eine vorteilhafte Wirkung auf die Anzahl der lebenden Zellen aufweist. Ferner ist erkennbar, dass die in Medium 1 enthaltene Eisenquelle (Eisen-Citrat-Diphosphat-Natrium-Komplex) gegenüber den in den Medien 5 bis 8 enthaltenen Eisenquellen nach 10 Tagen und nach 13 Tagen ab dem Beginn des Experiments eine vorteilhafte Wirkung auf die Produktkonzentration, d.h. auf die hergestellte Menge an Produkt, aufweist. Das Experiment belegt, dass sich der Eisen-Citrat-Diphosphat-Natrium-Komplex nicht in den flüssigen Medien aus seinen einzelnen Komponenten bildet, denn sonst müssten die erhaltenen Werte in den Medien 5 bis 8 zu den erhaltenen Werten für Medium 1 identisch sein. Dies war jedoch nicht der Fall. Es ist zudem belegt, dass der Eisen-Citrat-Diphosphat-Natrium-Komplex (in Medium 1) gegenüber einer Mischung aus Eisen(III)citrat und tetrabasischem Natriumpyrophosphat vorteilhaft auf die erhaltene Menge an hergestelltem Produkt ist. (vgl. Medium 1 und 5 in Figur 4B).

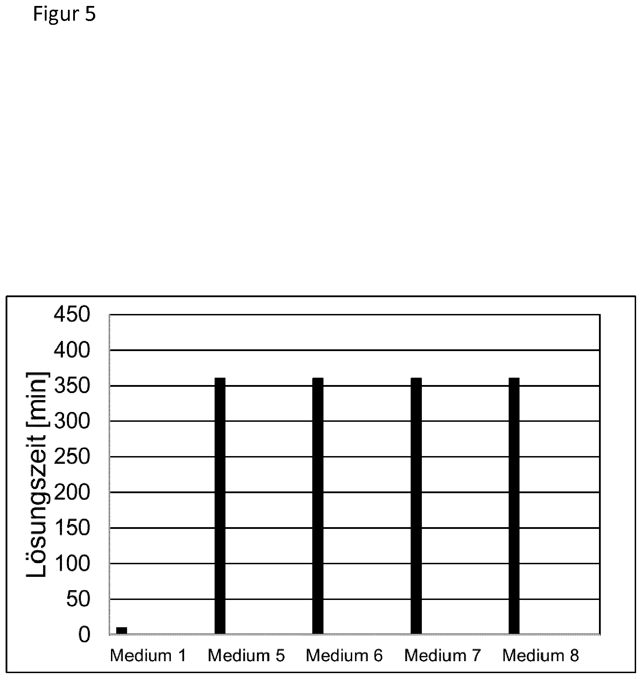

Figur 5 zeigt die Dauer bis zur vollständigen Lösung eines mit einem Eisen-Citrat-Diphosphat-Natrium-Komplex als Eisenquelle versetzten pulverförmigen Transferrin-freien Kulturmediums in Wasser im Vergleich zu Medien, welche die gleiche Zusammensetzung aufweisen und sich nur darin unterscheiden, dass die Komponenten des Eisen-Citrat-Diphosphat-Komplexes in Form von Einzelkomponenten vorliegen. Anders ausgedrückt sind die fünf auf der x-Achse dargestellten Medien 1, 5, 6, 7 und 8 in ihrer Zusammensetzung vergleichbar bis auf die Quelle von Eisen, Citrat und Pyrophosphat. Auf der y-Achse ist die Dauer (in Minuten) angegeben, bis sich das ursprünglich trockene, pulverförmige Medium vollständig in Wasser gelöst hat. Es ist erkennbar, dass sich das trockene, pulverförmige Medium 1 mit dem Eisen-Citrat-Diphosphat-Natrium-Komplex im Vergleich mit den Medien 5, 6, 7 und 8, welche die Einzelkomponenten des Eisen-Citrat-Diphosphat-Komplexes aufweisen (d.h. Salze zur Bereitstellung von Eisenionen, Citrationen, Pyrophosphationen und Natriumionen), deutlich schneller in Wasser löst, wobei mehr als 360 Minuten benötigt wurden, um eine vollständige Lösung der ursprünglich trockene, pulverförmige Medien 5, 6 und 7 zu erreichen und Medium 8 sich nicht vollständig löst.

### Beispiel - Biologische Zellen, die mit dem erfindungsgemäßen Zellkulturmedium kultiviert werden können

| Zelllinie | Beispiel für diese Zelllinie |
|---|---|
| NSO | ECACC No. 85110503 |
| Sp2/0-Ag14 | ATCC CRL-1581 |
| BHK21 | ATCC CCL-10 |
| BHK TK- | ECACC No. 85011423 |
| HaK | ATCC CCL-15 |
| 2254-62.2 (BHK-21 derivative) | ATCC CRL-8544 |
| CHO | ECACC No. 8505302 |
| CHO wild type | ECACC 00102307 |
| CHO-K1 | ATCC CCL-61 |
| CHO-DUKX (= CHO duk-, CHO/dhfr-) | ATCC CRL-9096 |
| CHO-DUKX B11 | ATCC CRL-9010 |
| CHO-DG44 | Urlaub et al., 1983 |
| CHO Pro-5 | ATCC CRL-1781 |
| CHO-S | Freedom^{™} CHO-S^{™} Kit, Thermo |
| V79 | Fisher Scientific Cat no. R800-07 ATCC CCC-93 |
| B14AF28-G3 | ATCC CCL-14 |
| PER.C6 | (Fallaux, F.J. et al, 1998) |
| HEK 293 | ATCC CRL-1573 |
| COS-7 | ATCC CRL-1651 |
| U266 | ATCC TIB-196 |
| HuNS1 | ATCC CRL-8644 |
| CHL | ECACC No. 87111906 |
| PER-C6 | |
| human liver cells | Hep G2, HB 8065 |
| human lung cells | W138, ATCC CCL 75 |
| human cervical Carcinoma cells | (HeLa, ATCC CCL 2) |
| monkey kidney cells | COS- 7, ATCC CRL 1651 |
| canine kidney cells | MDCK |
| monkey kidney cells | CV1, ATCC CCL 70 |
| afrikan green monkey kidney cells | VERO-76, ATCC CRL-1587 |
| baby hamster kidney cells | BHK-21, ATCC CCL 10 |
| Chinese hamster ovary cells | CHO-DG44 |
| CHO- DUKX | |
| CHO-K1 | ATCC CCL 61 |
| lym- phocytic cells | Jurkat T-cell line) |
| buffalo rat liver cells | BRL 3A, ATCC CRL 1442 |
| mouse mammary tumor cells | MMT 060562, ATCC CCL 51 |
| SP2/0 Zellen | |
| Myelomazellen | NSO |
| Hybridomazellen | |
| Triomazellen. | |

### Beispiel 2 - Ermittlung der optimalen Konzentration des Eisen-Citrat-Diphosphat-Komplex im Zellkulturmedium

Um den optimalen Konzentrationsbereich für den Eisen-Citrat-Diphosphat-Komplex im Zellkulturmedium festzustellen wurde ein Eisen-Citrat-Diphosphat-Natrium-Komplex in unterschiedlichen Konzentrationen in einem Transferrin-freien Zellkulturmedium getestet.

Das verwendete Zellkulturmedium basierte auf einem Dulbecco's Modified Eagle's Medium/Ham's nutrient Mixture F-12 (DMEM/F12-Medium). Es enthielt Spurenelemente und Salze der Elemente Calcium, Eisen, Kobalt, Kupfer, Kalium, Magnesium, Mangan, Molybdän, Natrium, Nickel, Phosphat, Selen, Silizium, Zink und Zinn. Die Eisenkonzentration in dem verwendeten Zellkulturmedium ohne die Zugabe des Eisen-Citrat-Diphosphat-Komplexes betrug unter 0,8 µM. Ferner enthielt das verwendete Zellkulturmedium Glycin, L-Alanin, L-Arginin, L-Asparagin, L-Asparaginsäure, L-Cystein, L-Cystin, L-Glutaminsäure, L-Glutamin, L-Histidin, L-Isoleucin, L-Leucin, L-Lysin, L-Methionin, L-Phenylalanin, L-Prolin, L-Serin, L-Threonin, L-Tryptophan, L-Tyrosin und L-Valin. Zudem enthielt das verwendete Zellkulturmedium Biotin, Cholin, Folinsäure, Glukose, Hepes-Puffer, Hypoxanthin, Linolsäure, Liponsäure, Myoinositol, Niacinamid, Pantothensäure, Putrescin, Pyridoxal, Pyridoxin, Riboflavin, Thiamin, Tymidin, Pyruvat und Vitamin B12.

Es wurde das Zellwachstum von Zellen, die gentechnisch zur Herstellung eines bestimmten Proteins verändert wurden, in Abhängigkeit von der über den Eisen-Citrat-Diphosphat-Natrium-Komplex eingestellten Eisen-Konzentration in dem in Wasser gelösten, auf dem oben genannten DMEM/F12-Mediumbasierten, flüssigen Zellkulturmedium untersucht. Zusätzlich wurde die Herstellung des gewünschten Proteinprodukts in Abhängigkeit von der über den Eisen-Citrat-Diphosphat-Natrium-Komplex eingestellten Eisen-Konzentration in dem flüssigen Medium untersucht.

Die Ergebnisse auf das Zellwachstum und die Proteinproduktion in Abhängigkeit von der über den Eisen-Citrat-Diphosphat-Natrium-Komplex eingestellten Eisen-Konzentration sind in Figur 1A und 1B dargestellt. Es zeigte sich, dass eine finale Eisenkonzentration im Bereich von ca. 80 µM bis 5800 µM in dem flüssigen Medium ein Optimum auf das Zellwachstum und die Proteinproduktion darstellt. Da die Eisenkonzentration in dem verwendeten Zellkulturmedium ohne die Zugabe des Eisen-Citrat-Diphosphat-Komplexes unter 0,8 µM betrug, wurde diese finale Eisenkonzentration im Bereich von ca. 80 µm bis 5800 µm über den Einsatz einer praktisch entsprechenden molaren Menge des Eisen-Citrat-Diphosphat-Komplexes erzielt (d.h. ca. 80 µm bis 5800 µm Eisen-Citrat-Diphosphat-Natrium-Komplex).

### Beispiel 3 - Einfluss von Eisen-Citrat-Diphosphat-Natrium-Komplex im Vergleich zu anderen Eisenquellen auf das Wachstum und die Proteinproduktion der Zellen

Es wurden insgesamt vier verschiedene Medien getestet, die bis auf die eingesetzte Eisenquelle dem in Beispiel 2 aufgeführten Transferrin-freien Kulturmedium entsprachen und somit bis auf die eingesetzte Eisenquelle eine identische Zusammensetzung aufwiesen. Die eingesetzte Eisenquelle wurde allen Medien in einer Menge zugefügt, sodass eine finale Eisenkonzentration von 300 µM im flüssigen Medium erreicht wurde.

Als Eisenquelle wurde eingesetzt:

| | |
|---|---|
| Medium 1: | Eisen-Citrat-Diphosphat-Natrium-Komplex (300 µM Eisen-Konzentration); |
| Medium 2: | Eisen(II)sulfat-Heptahydrat (300 µM Eisen-Konzentration); |
| Medium 3: | Eisen(III)citrat-Nonahydrat (300 µM Eisen-Konzentration); |
| Medium 4: | Eisen(III)citrat (300 µM Eisen-Konzentration). |

Es wurde der Einfluss auf das Zellwachstum und die Proteinproduktion von den kultivierten Zellen, die gentechnisch zur Herstellung eines bestimmten Proteins verändert wurden, nach 10 Tagen und nach 13 Tagen ab dem Beginn des Zellkultur-Experiments (Fed-Batch) untersucht. Das Ergebnis ist in Figur 2A und 2B dargestellt. Es zeigte sich, dass die in Medium 1 enthaltene Eisenquelle (Eisen-Citrat-Diphosphat-Natrium-Komplex) gegenüber den in den Medien 2 bis 4 enthaltenen Eisenquellen nach 10 Tagen und nach 13 Tagen ab dem Beginn des Experiments eine vorteilhafte Wirkung auf die Anzahl der lebenden Zellen als auch auf die Produktkonzentration, d.h. auf die hergestellte Menge an Produkt, aufweist.

### Beispiel 4 - Löslichkeit von einem trockenen Medium mit der Eisenquelle Eisen-Citrat-Diphosphat-Natrium-Komplex in Wasser im Vergleich zu trockenen Medien mit anderen Eisenquellen

Es wurden die Medien aus Beispiel 3 in trockener, pulverförmiger Form mit Wasser versetzt und die Zeit gemessen, bis die trockenen, pulverförmigen Medien jeweils vollständig in Lösung gegangen sind. Die Ergebnisse sind in Figur 3 dargestellt. Es ist erkennbar, dass sich das trockene, pulverförmige Medium 1 mit dem Eisen-Citrat-Diphosphat-Natrium-Komplex als Eisenquelle gegenüber den Medien 2, 3 und 4, die eine andere Eisenquelle aufweisen, deutlich schneller in Wasser löst, wobei mehr als 360 Minuten benötigt wurden, um eine vollständige Lösung der ursprünglich trockene, pulverförmige Medien 2, 3 und 4 zu erreichen.

### Beispiel 5 - Einfluss von Eisen-Citrat-Diphosphat-Natrium-Komplex im Vergleich zu anderen Eisenquellen, die zusätzlich Einzelkomponenten des Eisen-Citrat-Diphosphat-Natrium-Komplexes aufweisen, auf das Wachstum und die Proteinproduktion der Zellen

Es wurden insgesamt fünf verschiedene Medien getestet, die bis auf die eingesetzte Eisenquelle dem in Beispiel 2 aufgeführten Transferrin-freien Kulturmedium entsprachen und bis auf die eingesetzte Eisenquelle und bestimmte Einzelkomponenten des Eisen-Citrat-Diphosphat-Natrium-Komplexes eine identische Zusammensetzung aufwiesen. Die eingesetzte Eisenquelle wurde allen Medien in einer Menge zugefügt, sodass eine finale Eisenkonzentration von 300 µM im flüssigen Medium erreicht wurde. Die zusätzlichen Einzelkomponenten des Eisen-Citrat-Diphosphat-Natrium-Komplexes aus Medium 1, die eine Bildung des Komplexes in dem gelösten Medium erlauben sollten, wurden den Vergleichsmedien 5, 6, 7 und 8 in möglichst äquimolaren Mengen zugeführt. Zu Berechnung der äquimolaren Mengen von Pyrophosphat und Citrat wurde ein Eisen zu Citrat zu Pyrophosphate Verhältnis von 4 zu 3 zu 3 benutzt welches durch Röntgenabsorptionsspektroskopie gemäß Gupta et al. (Physicochemical characterization of ferric pyrophosphate citrate, Biometals, 2018, Bd. 31, S. 1091-1099.) ermittelt wurde. Anders ausgedrückt wurde die Menge an Pyrophosphat und Citrat in den Medien 5 bis 8 vergleichbar zu der durch den Eisen-Citrat-Diphosphat-Natrium-Komplexes bereitgestellten Konzentration von Pyrophosphat und Citrat in Medium 1 gehalten. Ziel war die Untersuchung, ob die Einzelkomponenten des Eisen-Citrat-Diphosphat-Natrium-Komplexes eine vergleichbare Wirkung wie der Komplex aufweist beziehungsweise ob sich der Komplex eventuell spontan aus den Einzelkomponenten in wässriger Lösung bilden kann.

Die fünf getesteten Medien enthielten als Eisenquelle, Pyrophosphatquelle und Citratquelle:

| | |
|---|---|
| Medium 1: | Eisen-Citrat-Diphosphat-Natrium-Komplex (300 µM Eisen-Konzentration); |
| Medium 5: | Eisen(III)citrat (300 µM Eisen-Konzentration) tetrabasisches Natriumpyrophosphat (225 µM PyrophosphatKonzentration); |
| Medium 6: | Eisen(II)sulfat-Heptahydrat (300 µM Eisen-Konzentration) tetrabasisches Natriumpyrophosphat (225 µM PyrophosphatKonzentration) tribasisches Natriumcitrat-Dihydrat (225 µM CitratKonzentration) |
| Medium 7: | Eisen(III)nitrat-Nonahydrat (300 µM Eisen-Konzentration) tetrabasisches Natriumpyrophosphat (225 µM Pyrophosphate-Konzentration) tribasisches Natriumcitrat-Dihydrat (225 µM CitratKonzentration) |
| Medium 8: | Eisen(III)pyrophosphat (300 µM Eisen-Konzentration) tribasisches Natriumcitrat-Dihydrat (225 µM CitratKonzentration) |

Es wurde der Einfluss auf das Zellwachstum und die Proteinproduktion von den kultivierten Zellen, die gentechnisch zur Herstellung eines bestimmten Proteins verändert wurden, nach 10 Tagen und nach 13 Tagen ab dem Beginn des Zellkultur-Experiments (Fed-Batch) untersucht. Das Ergebnis ist in Figur 4A und 4B dargestellt. Es zeigte sich, dass die in Medium 1 enthaltene Eisenquelle (Eisen-Citrat-Diphosphat-Natrium-Komplex) gegenüber den in den Medien 6 bis 8 enthaltenen Eisenquellen nach 10 Tagen und nach 13 Tagen ab dem Beginn des Experiments eine vorteilhafte Wirkung auf die Anzahl der lebenden Zellen aufweist. Ferner ist erkennbar, dass die in Medium 1 enthaltene Eisenquelle (Eisen-Citrat-Diphosphat-Natrium-Komplex) gegenüber den in den Medien 5 bis 8 enthaltenen Eisenquellen nach 10 Tagen und nach 13 Tagen ab dem Beginn des Experiments eine vorteilhafte Wirkung auf die Produktkonzentration, d.h. auf die hergestellte Menge an Produkt, aufweist.

Das Experiment belegt, dass sich der Eisen-Citrat-Diphosphat-Natrium-Komplex nicht in den flüssigen Medien aus seinen einzelnen Komponenten bildet, denn sonst müssten die Ergebnisse für die Medien 5 bis 8 zu dem Ergebnis für Medium 1 identisch sein. Dies war jedoch nicht der Fall.

Ferner geht aus den Daten hervor, dass der Eisen-Citrat-Diphosphat-Natrium-Komplex (Medium 1) gegenüber einer Mischung aus Eisen(III)citrat und tetrabasischem Natriumpyrophosphat (Medium 5) vorteilhaft auf die erhaltene Menge an hergestelltem Produkt ist. (vgl. Medium 1 und 5 in Figur 4B). Da das Medium 5 Pyrophosphat in freier, anionischer Form und nicht in komplexierter Form (im Eisen-Citrat-Diphosphat-Natrium-Komplex) enthält, könnte dies bedeuten, dass für die Zellen in dem Eisen-Citrat-Diphosphat-Natrium-Komplex gebundenes Pyrophosphat besser zugänglich ist, d.h. leichter aufgenommen werden kann als freies, anionisches Pyrophosphat und somit die über den Eisen-Citrat-Diphosphat-Natrium-Komplex erreichbare intrazelluläre Pyrophosphatkonzentration höher ist als mit freiem Pyrophosphat im Medium möglich ist.

Die Medien 5 bis Medium 8 wurden angesetzt um zu zeigen, dass Eisen-Komplex nicht spontan selbst bildet und es einen Performance-Unterschied gibt.

### Beispiel 6 - Löslichkeit von einem trockenen Medium mit dem Eisen-Citrat-Diphosphat-Natrium-Komplex in Wasser im Vergleich zu trockenen Medien mit anderen Einzelkomponenten des Komplexes (d.h. Eisen, Citrat und Pyrophosphat als Einzelkomponenten)

Es wurden die Medien aus Beispiel 5 in trockener, pulverförmiger Form mit Wasser versetzt und die Zeit gemessen, bis die trockenen, pulverförmigen Medien jeweils vollständig in Lösung gegangen sind. Die Ergebnisse sind in Figur 5 dargestellt. Es wird deutlich, dass sich das trockene, pulverförmige Medium 1 mit dem Eisen-Citrat-Diphosphat-Natrium-Komplex im Vergleich mit den Medien 5, 6, 7 und 8, welche die Einzelkomponenten des Eisen-Citrat-Diphosphat-Komplexes aufweisen (d.h. Salze zur Bereitstellung von Eisenionen, Citrationen, Pyrophosphationen und Natriumionen), deutlich schneller in Wasser löst, wobei mehr als 360 Minuten benötigt wurden, um eine vollständige Lösung der ursprünglich trockene, pulverförmige Medien 5, 6 und 7 zu erreichen und Medium 8 sich nicht vollständig löst.

## Patentansprüche

1. Zellkulturmedium , **dadurch gekennzeichnet, dass** das Zellkulturmedium einen Eisen-Citrat-Diphosphat-Komplex enthält und in ungelöster Form vorliegt, wobei das Zellkulturmedium den Eisen-Citrat-Diphosphat-Komplex in einer Menge von 0,16 Gew.-% bis 12 Gew.-% enthält.

2. Zellkulturmedium, **dadurch gekennzeichnet, dass** das Zellkulturmedium einen Eisen-Citrat-Diphosphat-Komplex enthält und in gelöster Form vorliegt, wobei das gelöste Zellkulturmedium den Eisen-Citrat-Diphosphat-Komplex in einer Menge enthält, dass die über den Eisen-Citrat-Diphosphat-Komplex eingestellte Eisenkonzentration des Zellkulturmediums im Bereich von 80 µM bis 5800 µM liegt, wobei der Eisen-Citrat-Diphosphat-Komplex ein Eisen-Citrat-Diphosphat-Natrium-Komplex mit der CAS Nr. 85338-24-5 ist.

3. Zellkulturmedium nach einem der vorhergehenden Ansprüche, **dadurch gekennzeichnet, dass** das Zellkulturmedium
i) frei von mindestens einem Serumbestandteil, bevorzugt frei von Transferrin und/oder Lactotransferin, ist; und/oder
ii) einen Serumersatz enthält, bevorzugt einen Ultroser G-Serumersatz in der im August 2018 erhältlichen Zusammensetzung; und/oder
iii) Wachstumfaktoren enthält; und/oder
iv) frei von tierischen oder humanen Komponenten ist; und/oder
v) proteinfrei ist; und/oder
vi) hydrolysatfrei ist; und/oder
vii) chemisch-definiert ist.

4. Zellkulturmedium nach einem der vorhergehenden Ansprüche, **dadurch gekennzeichnet, dass** das Zellkulturmedium
i) mindestens eine, bevorzugt mehrere, Aminosäure(n) enthält, wobei die mindestens eine, bevorzugt mehrere, Aminosäure(n) bevorzugt ausgewählt ist/sind aus der Gruppe bestehend aus Alanin, Arginin, Asparagin, Asparaginsäure, Cystein, Cystine, Glutamin, Glutaminsäure, Glycin, Histidin, Isoleucin, Leucin, Lysin, Methionin, Phenylalanin, Prolin, Serin, Threonin, Tryptophan, Tyrosin, Valin und Kombinationen und/oder Salze hiervon, wobei das Zellkulturmedium insbesondere alle dieser Aminosäuren enthält; und/oder
ii) mindestens eine, bevorzugt mehrere, Lipid-Vorstufe(n) enthält, wobei die mindestens eine, bevorzugt mehrere Lipid-Vorstufe(n) bevorzugt ausgewählt sind aus der Gruppe bestehend aus Cholinchlorid, Ethanolamine, Glycerin, Inositol, Linolensäure, Fettsäure, Phopholipid, Cholesterin-verwandte Verbindungen und Kombinationen und Salze hiervon; und/oder
iii) mindestens eine, bevorzugt mehrere, Carbonsäure(n) mit mindestens sechs C-Atomen enthält, wobei die mindestens eine, bevorzugt mehreren, Carbonsäure(n) bevorzugt ausgewählt ist/sind aus der Gruppe bestehend aus Linolsäure, Linolensäure, Thioctsäure, Ölsäure, Palmitinsäure, Stearinsäure, Arachinsäure, Arachidonsäure, Laurinsäure, Behensäure, Decansäure, Dodecansäure, Hexansäure, Lignocerinsäure, Myristinsäure, Octansäure und Kombinationen und Salze hiervon, wobei das Zellkulturmedium insbesondere alle dieser Fettsäuren und/oder Salze hiervon enthält; und/oder
iv) mindestens eine, bevorzugt mehrere, Carbonsäure(n) mit weniger als sechs C-Atomen enthält, wobei die mindestens Carbonsäure bevorzugt Buttersäure oder ein Salz der Buttersäure ist; und/oder
v) mindestens ein, bevorzugt mehrere, Nukleosid(e) enthält, wobei das mindestens eine, bevorzugt mehrere, Nukleosid(e) ausgewählt sind aus der Gruppe bestehend aus Adenosin, Guanosin, Cytidin, Uridin, Thymidin, Hypoxanthin und Kombinationen und Salze hiervon, wobei das Zellkulturmedium insbesondere alle dieser Nukleoside und/oder Salze hiervon enthält; und/oder
vi) mindestens ein, bevorzugt mehrere, Kohlenhydrat(e), enthält, wobei das mindestens eine, bevorzugt mehrere, Kohlenhydrat(e) bevorzugt ausgewählt ist/sind aus der Gruppe bestehend aus Glucose, Galactose, Glucosamin, Fructose, Mannose, Ribose, Saccharose und Kombinationen hiervon.

5. Zellkulturmedium nach einem der vorhergehenden Ansprüche, **dadurch gekennzeichnet, dass** das Zellkulturmedium
i) mindestens eine, optional mehrere, Puffersubstanz(en) enthält, wobei mindestens eine, optional mehrere, Puffersubstanz(en) bevorzugt ausgewählt ist/sind aus der Gruppe bestehend aus ACES, HEPES, MES, MOPS, NaHCO₃, PIPES, Phosphat-Puffer, TRIS und Kombinationen und/oder Salze hiervon; und/oder
ii) mindestens ein, bevorzugt mehrere Spurenelement(e) enthält, optional ferner einen Chelatbildner, bevorzugt EDTA, enthält, wobei das mindestens eine, bevorzugt mehrere, Spurenelement(e) bevorzugt ausgewählt ist/sind aus der Gruppe bestehend aus Calcium, Kalium, Kobalt, Kupfer, Magnesium, Mangan, Molybdän, Natrium, Nickel, Phosphat, Selen, Vanadium, Zink, Zinn und Kombinationen hiervon, wobei das Spurenelement optional in Salzform vorliegen kann und wobei das Zellkulturmedium insbesondere alle dieser Spurenelemente enthält; und/oder
iii) mindestens ein, bevorzugt mehrere Vitamin(e) enthält, wobei das mindestens eine, bevorzugt mehrere, Vitamin(e) bevorzugt ausgewählt ist/sind aus der Gruppe bestehend aus Biotin, Cholin, Folinsäure, Myoinositol, Niacinamid (B3), Pantothensäure, Pyridoxal, Pyridoxin, Riboflavin, Thiamin, Vitamin B12 and/or und Kombinationen und/oder Salze hiervon enthält.

6. Zellkulturmedium nach einem der vorhergehenden Ansprüche, **dadurch gekennzeichnet, dass** das Zellkulturmedium, besonders bevorzugt in ungelöster Form,
i) kein Eisensalz, enthält; und/oder
ii) kein Eisencitrat, bevorzugt kein Citratsalz und/oder keine Citronensäure, enthält; und/oder
iii) kein Eisendiphosphat, bevorzugt kein Diphosphatsalz und/oder keine Diphosphorsäure, enthält.

7. Zellkulturmedium nach einem der vorhergehenden Ansprüche, **dadurch gekennzeichnet, dass** das Zellkulturmedium ein Zellkulturmedium ausgewählt aus der Gruppe bestehend aus DMEM, DMEM/F12 Media, Ham's F-10 Media, Ham's F-12 Media, Medium 199, MEM, RPMI 1640 Medium, ISF-1, Octomed, Ames' Medium, BGJb Medium (optional in der Fitton-Jackson Modification), Click's Medium, CMRL-1066 Medium, Fischer's Medium, Glascow Minimum Essential Medium (GMEM), Iscove's Modified Dulbecco's Medium (IMDM), L-15 Medium (Leibovitz), McCoy's 5A Modified Medium, NCTC Medium, Swim's S-77 Medium, Waymouth Medium, William's Medium E und Kombinationen hiervon, optional auch Modifikationen hiervon, enthält, wobei das jeweilige Zellkulturmedium in der im August 2018 erhältlichen Zusammensetzung gemeint ist.

8. Zellkulturmedium nach dem vorhergehenden Anspruch, **dadurch gekennzeichnet, dass** das Zellkulturmedium ein Zellkulturmedium ausgewählt aus der Gruppe bestehend aus ein Zellkulturmedium ausgewählt aus der Gruppe bestehend aus DMEM, DMEM/F12 Media, Ham's F-10 Media, Ham's F-12 Media, Medium 199, MEM, RPMI 1640 Medium und Kombinationen hiervon, optional auch Modifikationen hiervon, enthält, wobei das jeweilige Zellkulturmedium in der im August 2018 erhältlichen Zusammensetzung gemeint ist.

9. Verfahren zum Kultivieren von Zellen, umfassend die Schritte:
a) Bereitstellen eines Zellkulturmediums in einer wässrigen Lösung, wobei das gelöste Zellkulturmedium einen Eisen-Citrat-Diphosphat-Komplex in einer Menge enthält, dass die über den Eisen-Citrat-Diphosphat-Komplex eingestellte Eisenkonzentration des Zellkulturmediums im Bereich von 80 µM bis 5800 µM liegt, wobei der Eisen-Citrat-Diphosphat-Komplex ein Eisen-Citrat-Diphosphat-Natrium-Komplex mit der CAS Nr. 85338-24-5 ist; und
b) Vermehren oder Halten mindestens einer Zelle in der wässrigen Lösung des Zellkulturmediums.

10. Verfahren nach Anspruch 9, wobei die Zelle eine Zelle einer Primärzelllinie oder einer kontinuierliche Zelllinie ist und bevorzugt ausgewählt ist aus der Gruppe bestehend aus Säugetierzelle, Vogelzelle und Insektenzelle, wobei die Zelle besonders bevorzugt eine Säugetierzelle ist, besonders bevorzugt eine Säugetierzelle ausgewählt aus der Gruppe bestehend aus CHO, NS0, SP2/0, Hybridoma, HEK293, PERC-6, BHK-21 und Vero-76, ganz besonders bevorzugt eine CHO-Zelle, insbesondere eine CHO-Zelle ausgewählt aus der Gruppe bestehend aus CHO-DG44, CHO-DUKX, CHO-S und CHO-K1.

11. Verfahren zur Herstellung von mindestens einem rekombinanten Protein in einer Zellkultur, wobei das Verfahren nach einem der Ansprüche 9 oder 10 ferner ein Einführen einer Nukleinsäure in die mindestens eine Zelle umfasst, wobei die Nukleinsäure eine konstitutive oder induzierte Produktion von mindestens einem rekombinanten Protein bewirkt, bevorzugt zusätzlich eine Sezernierung des produzierten Proteins in die wässrige Lösung des Zellkulturmediums bewirkt, wobei das rekombinante Protein besonders bevorzugt ausgewählt ist aus der Gruppe bestehend aus therapeutisches Protein, Antikörper, Fusionsprotein, Enzym, Vakzin, Biosimilar und Kombinationen hiervon, bevorzugt ausgewählt aus der Gruppe bestehend aus Kinase, Hormon, Antikörper, Vakzin, Reporterprotein-Fusionsprotein und Kombinationen hiervon.

12. Verwendung eines Eisen-Citrat-Diphosphat-Komplexes mit der CAS Nr. 85338-24-5 in einem Zellkulturmedium, wobei das Zellkulturmedium in gelöster Form vorliegt und einen Eisen-Citrat-Diphosphat-Komplex in einer Menge enthält, dass die über den Eisen-Citrat-Diphosphat-Komplex eingestellte Eisenkonzentration des Zellkulturmediums im Bereich von 80 µM bis 5800 µM liegt.

## Claims

1. Cell culture medium, **characterised in that** the cell culture medium comprises an iron-citrate-diphosphate complex and is present in undissolved form, the cell culture medium comprising the iron-citrate-diphosphate complex in a quantity of from 0.16 wt.-% to 12 wt.-%.

2. Cell culture medium, **characterised in that** the cell culture medium comprises an iron-citrate-diphosphate complex and is present in dissolved form, the dissolved cell culture medium comprising the iron-citrate-diphosphate complex in a quantity such that the iron concentration of the cell culture medium set by the iron-citrate-diphosphate complex is in the range of from 80 µM to 5800 µM, the iron-citrate-diphosphate complex being an iron-citrate-diphosphate-sodium complex having the CAS No. 85338-24-5.

3. Cell culture medium according to any of the preceding claims, **characterised in that** the cell culture medium
i) is free of at least one serum constituent, preferably free of transferrin and/or lactotransferrin; and/or
ii) comprises a serum substitute, preferably an Ultroser-G serum substitute having the composition available in August 2018; and/or
iii) comprises growth factors; and/or
iv) is free of animal or human components; and/or
v) is free of proteins; and/or
vi) is free of hydrolysates; and/or
vii) is chemically defined.

4. Cell culture medium according to any of the preceding claims, **characterised in that** the cell culture medium
i) comprises at least one, preferably a plurality of, amino acid(s), the at least one, preferably a plurality of, amino acid(s) preferably being selected from the group consisting of alanine, arginine, asparagine, aspartic acid, cysteine, cystine, glutamine, glutamic acid, glycine, histidine, isoleucine, leucine, lysine, methionine, phenylalanine, proline, serine, threonine, tryptophan, tyrosine, valine and combinations and/or salts thereof, the cell culture medium in particular comprising all of these amino acids; and/or
ii) comprises at least one, preferably a plurality of, lipid precursor(s), the at least one, preferably a plurality of, lipid precursor(s) preferably being selected from the group consisting of choline chloride, ethanolamines, glycerol, inositol, linolenic acid, fatty acid, phospholipid, cholesterol-related compounds and combinations and salts thereof; and/or
iii) comprises at least one, preferably a plurality of, carboxylic acid(s) having at least six C atoms, the at least one, preferably a plurality of, carboxylic acid(s) preferably being selected from the group consisting of linoleic acid, linolenic acid, thioctic acid, oleic acid, palmitic acid, stearic acid, arachidic acid, arachidonic acid, lauric acid, behenic acid, decanoic acid, dodecanoic acid, hexanoic acid, lignoceric acid, myristic acid, octanoic acid and combinations and salts thereof, the cell culture medium in particular comprising all of these fatty acids and/or salts thereof; and/or
iv) comprises at least one, preferably a plurality of, carboxylic acid(s) having less than six C atoms, the at least one carboxylic acid preferably being butyric acid or a salt of butyric acid; and/or
v) comprises at least one, preferably a plurality of, nucleoside(s), the at least one, preferably a plurality of, nucleoside(s) preferably being selected from the group consisting of adenosine, guanosine, cytidine, uridine, thymidine, hypoxanthine and combinations and salts thereof, the cell culture medium in particular comprising all of these nucleoside and/or salts thereof; and/or
vi) comprises at least one, preferably a plurality of, carbohydrate(s), the at least one, preferably a plurality of, carbohydrate(s) preferably being selected from the group consisting of glucose, galactose, glucosamine, fructose, mannose, ribose, sucrose and combinations thereof.

5. Cell culture medium according to any of the preceding claims, **characterised in that** the cell culture medium
i) comprises at least one, optionally a plurality of, buffer substance(s), at least one, optionally a plurality of, buffer substance(s) preferably being selected from the group consisting of ACES, HEPES, MES, MOPS, NaHCO₃, PIPES, phosphate buffer, TRIS and combinations and/or salts thereof; and/or
ii) comprises at least one, preferably a plurality of, trace element(s), optionally also a chelating agent, preferably EDTA, the at least one, preferably a plurality of, trace element(s) preferably being selected from the group consisting of calcium, potassium, cobalt, copper, magnesium, manganese, molybdenum, sodium, nickel, phosphate, selenium, vanadium, zinc, tin and combinations thereof, the trace element optionally being able to be in salt form and the cell culture medium in particular comprising all of these trace elements; and/or
iii) comprises at least one, preferably a plurality of, vitamin(s), the at least one, preferably a plurality of, vitamin(s) preferably being selected from the group consisting of biotin, choline, folinic acid, myoinositol, niacinamide (B3), pantothenic acid, pyridoxal, pyridoxine, riboflavin, thiamine, vitamin B12 and/or combinations and/or salts thereof.

6. Cell culture medium according to any of the preceding claims, **characterised in that** the cell culture medium, particularly preferably in undissolved form,
i) does not contain any iron salt; and/or
ii) does not contain any iron citrate, preferably does not contain any citrate salt and/or citric acid; and/or
iii) does not contain any iron diphosphate, preferably does not contain any diphosphate salt and/or diphosphoric acid.

7. Cell culture medium according to any of the preceding claims, **characterised in that** the cell culture medium comprises a cell culture medium selected from the group consisting of DMEM, DMEM/F12 Medium, Ham's F-10 Medium, Ham's F-12 Medium, Medium 199, MEM, RPMI 1640 Medium, ISF-1, Octomed, Ames' Medium, BGJb Medium (optional in the Fitton-Jackson Modification), Click's Medium, CMRL-1066 Medium, Fischer's Medium, Glasgow Minimum Essential Medium (GMEM), Iscove's Modified Dulbecco's Medium (IMDM), L-15 Medium (Leibovitz), McCoy's 5A Modified Medium, NCTC Medium, Swim's S-77 Medium, Waymouth's Medium, William's Medium E and combinations thereof, optionally also modifications thereto, the relevant cell culture medium having the composition available in August 2018 being meant.

8. Cell culture medium according to the preceding claim, **characterised in that** the cell culture medium comprises a cell culture medium selected from the group consisting of a cell culture medium selected from the group consisting of DMEM, DMEM/F12 Medium, Ham's F-10 Medium, Ham's F-12 Medium, Medium 199, MEM, RPMI 1640 Medium and combinations thereof, optionally also modifications thereto, the relevant cell culture medium having the composition available in August 2018 being meant.

9. Method for cultivating cells, comprising the steps of:
a) providing a cell culture medium in an aqueous solution,
wherein the dissolved cell culture medium comprises an iron-citrate-diphosphate complex in a quantity such that the iron concentration of the cell culture medium set by the iron-citrate-diphosphate complex is in the range of from 80 µM to 5800 µM,
wherein the iron-citrate-diphosphate complex is an iron-citrate-diphosphate-sodium complex having the CAS No. 85338-24-5; and
b) multiplying or maintaining at least one cell in the aqueous solution of the cell culture medium.

10. Method according to claim 9, wherein the cell is a cell of a primary cell line or a continuous cell line and is preferably selected from the group consisting of mammalian cells, avian cells and insect cells, wherein the cell is particularly preferably a mammalian cell, particularly preferably a mammalian cell selected from the group consisting of CHO, NSO, SP2/0, hybridoma, HEK293, PERC-6, BHK-21 and Vero-76, more particularly preferably a CHO cell, in particular a CHO cell selected from the group consisting of CHO-DG44, CHO-DUKX, CHO-S and CHO-K1.

11. Method for preparing at least one recombinant protein in a cell culture, wherein the method according to either of claims 9 or 10 further comprises introducing a nucleic acid into the at least one cell, wherein the nucleic acid effects a constitutive or an induced production of at least one recombinant protein, preferably additionally effects a secretion of the produced protein into the aqueous solution of the cell culture medium, wherein the recombinant protein is particularly preferably selected from the group consisting of a therapeutic protein, antibody, fusion protein, enzyme, vaccine, biosimilar and combinations thereof, preferably selected from the group consisting of a kinase, hormone, antibody, vaccine, reporter-protein-fusion-protein and combinations thereof.

12. Use of an iron-citrate-diphosphate complex having the CAS No. 85338-24-5 in a cell culture medium, wherein the cell culture medium is present in dissolved form and comprises an iron-citrate-diphosphate complex in a quantity such that the iron concentration of the cell culture medium set by the iron-citrate-diphosphate complex is in the range of from 80 µM to 5800 µM.

## Revendications

1. Milieu de culture cellulaire, **caractérisé en ce que** le milieu de culture cellulaire contient un complexe fer-citrate-diphosphate et se présente sous forme non dissoute, dans lequel le milieu de culture cellulaire contient le complexe fer-citrate-diphosphate dans une quantité comprise entre 0,16 % en masse et 12 % en masse.

2. Milieu de culture cellulaire, **caractérisé en ce que** le milieu de culture cellulaire contient un complexe fer-citrate-diphosphate et se présente sous forme dissoute, dans lequel le milieu de culture cellulaire dissous contient le complexe fer-citrate-diphosphate dans une quantité telle que la concentration en fer du milieu de culture cellulaire déterminée par le complexe fer-citrate-diphosphate est comprise entre 80 µM et 5 800 µM, dans lequel le complexe fer-citrate-diphosphate est un complexe fer-citrate-diphosphate-sodium dont le numéro CAS est 85338-24-5.

3. Milieu de culture cellulaire selon l'une des revendications précédentes, **caractérisé en ce que** le milieu de culture cellulaire
i) est exempt d'au moins un composant sérique, de préférence exempt de transferrine et/ou de lactotransferrine ; et/ou
ii) contient un substitut de sérum, de préférence un substitut de sérum Ultroser G dans la composition disponible en août 2018 ; et/ou
iii) contient des facteurs de croissance ; et/ou
iv) est exempt de composants animaux ou humains ; et/ou
v) est exempt de protéines ; et/ou
vi) est exempt d'hydrolysat ; et/ou
vii) est chimiquement défini.

4. Milieu de culture cellulaire selon l'une des revendications précédentes, **caractérisé en ce que** le milieu de culture cellulaire
i) contient au moins un, de préférence plusieurs, acide(s) aminé(s), dans lequel l'au moins un, de préférence plusieurs, acide(s) aminé(s) est/sont de préférence choisi(s) dans le groupe constitué par l'alanine, l'arginine, l'asparagine, l'acide aspartique, la cystéine, la cystine, la glutamine, l'acide glutamique, la glycine, l'histidine, l'isoleucine, la leucine, la lysine, la méthionine, la phénylalanine, la proline, la sérine, la thréonine, le tryptophane, la tyrosine, la valine et les combinaisons et/ou sels de ceux-ci, dans lequel le milieu de culture cellulaire contient en particulier tous ces acides aminés ; et/ou
ii) contient au moins un, de préférence plusieurs, précurseur(s) lipidique(s), dans lequel l'au moins un, de préférence plusieurs, précurseur(s) lipidique(s) est/sont de préférence choisi(s) dans le groupe constitué par le chlorure de choline, les éthanolamines, le glycérol, l'inositol, l'acide linolénique, l'acide gras, le phospholipide, les composés apparentés au cholestérol et les combinaisons et sels de ceux-ci ; et/ou
iii) contient au moins un, de préférence plusieurs, acide(s) carboxylique(s) à au moins six atomes de carbone, dans lequel l'au moins un, de préférence plusieurs, acide(s) carboxylique(s) est/sont de préférence choisi(s) dans le groupe constitué par l'acide linoléique, l'acide alpha-linoléique, l'acide thioctique, l'acide oléique, l'acide palmitique, l'acide stéarique, l'acide arachidique, l'acide arachidonique, l'acide laurique, l'acide béhénique, l'acide décanoïque, l'acide dodécanoïque, l'acide hexanoïque, l'acide lignocérique, l'acide myristique, l'acide octanoïque et les combinaisons et sels de ceux-ci, dans lequel le milieu de culture cellulaire contient en particulier tous ces acides gras et/ou les sels de ceux-ci ; et/ou
iv) contient au moins un, de préférence plusieurs, acide(s) carboxylique(s) à moins de six atomes de carbone, dans lequel l'au moins un acide carboxylique est de préférence l'acide butyrique ou un sel de l'acide butyrique ; et/ou
v) **caractérisé en ce que** le milieu de culture cellulaire contient au moins un, de préférence plusieurs, nucléoside(s), dans lequel l'au moins un, de préférence plusieurs, nucléoside(s) est/sont choisi(s) dans le groupe constitué par l'adénosine, la guanosine, la cytidine, l'uridine, la thymidine, l'hypoxanthine et les combinaisons et sels de celles-ci, dans lequel le milieu de culture cellulaire contient en particulier tous ces nucléosides et/ou les sels de ceux-ci ; et/ou
vi) contient au moins un, de préférence plusieurs, glucides, dans lequel l'au moins un, de préférence plusieurs, glucide(s) est/sont de préférence choisi(s) dans le groupe constitué par le glucose, le galactose, la glucosamine, le fructose, le mannose, le ribose, le saccharose et les combinaisons de ceux-ci.

5. Milieu de culture cellulaire selon l'une des revendications précédentes, **caractérisé en ce que** le milieu de culture cellulaire
i) contient au moins une, éventuellement plusieurs, substance(s) tampon(s), dans lequel l'au moins une, éventuellement plusieurs, substance(s) tampon(s) est/sont de préférence choisie(s) dans le groupe constitué par ACES, HEPES, MES, MOPS, NaHCO₃, PIPES, le tampon phosphate, TRIS et les combinaisons et/ou sels de ceux-ci ; et/ou
ii) contient au moins un, de préférence plusieurs, oligo-élément(s), contient éventuellement en outre un agent chélateur, de préférence l'EDTA, dans lequel l'au moins un, de préférence plusieurs, oligo-élément(s) est/sont de préférence choisi(s) dans le groupe constitué par le calcium, le potassium, le cobalt, le cuivre, le magnésium, le manganèse, le molybdène, le sodium, le nickel, le phosphate, le sélénium, le vanadium, le zinc, l'étain et les combinaisons de ceux-ci, dans lequel l'oligo-élément peut éventuellement se présenter sous forme de sel et dans lequel le milieu de culture cellulaire contient en particulier tous ces oligo-éléments ; et/ou
iii) contient au moins une, de préférence plusieurs, vitamine(s), dans lequel l'au moins une, de préférence plusieurs, vitamine(s) est/sont de préférence choisie(s) dans le groupe constitué par la biotine, la choline, l'acide folinique, le myo-inositol, la niacinamide (B3), l'acide pantothénique, le pyridoxal, la pyridoxine, la riboflavine, la thiamine, la vitamine B12 et les combinaisons et/ou sels de ceux-ci.

6. Milieu de culture cellulaire selon l'une des revendications précédentes, **caractérisé en ce que** le milieu de culture cellulaire, notamment de préférence sous forme non dissoute,
i) ne contient pas de sel de fer ; et/ou
ii) ne contient pas de citrate de fer, de préférence pas de sel de citrate et/ou pas d'acide citrique ; et/ou
iii) ne contient pas de diphosphate de fer, de préférence pas de sel de diphosphate et/ou pas d'acide diphosphorique.

7. Milieu de culture cellulaire selon l'une des revendications précédentes, **caractérisé en ce que** le milieu de culture cellulaire comprend un milieu de culture cellulaire choisi dans le groupe constitué par le DMEM, les milieux DMEM/F12, les milieux F-10 de Ham, les milieux F-12 de Ham, le milieu 199, le MEM, le milieu RMMI 1640, l'ISF-1, l'Octomed, le milieu d'Ames, le milieu BGJb (éventuellement dans la modification de Fitton-Jackson), le milieu de Click, le milieu CMRL-1066, le milieu de Fischer, le milieu essentiel minimum de Glascow (GMEM), le milieu de Dulbecco modifié selon Iscove (IMDM), le milieu L-15 (Leibovitz), le milieu modifié 5A de McCoy, le milieu NCTC, le milieu S-77 de Swim, le milieu de Waymouth, le milieu E de William et les combinaisons de ceux-ci, y compris éventuellement leurs modifications, dans lequel chaque milieu de culture cellulaire s'entend dans la composition disponible en août 2018.

8. Milieu de culture cellulaire selon la revendication précédente, **caractérisé en ce que** le milieu de culture cellulaire comprend un milieu de culture cellulaire choisi dans le groupe constitué par le DMEM, les milieux DMEM/F12, les milieux F-10 de Ham, les milieux F-12 de Ham, le milieu 199, le MEM, le milieu RMMI 1640 et les combinaisons de ceux-ci, y compris éventuellement leurs modifications, dans lequel chaque milieu de culture cellulaire s'entend dans la composition disponible en août 2018.

9. Procédé de culture de cellules comprenant les étapes suivantes :
a) obtention d'un milieu de culture cellulaire dans une solution aqueuse, dans lequel le milieu de culture cellulaire dissous contient un complexe fer-citrate-diphosphate dans une quantité telle que la concentration en fer du milieu de culture cellulaire déterminée par le complexe fer-citrate-diphosphate est comprise entre 80 µM et 5 800 µM,
dans lequel le complexe fer-citrate-diphosphate est un complexe fer-citrate-diphosphate-sodium dont le numéro CAS est 85338-24-5 ; et
b) multiplication ou maintien d'au moins une cellule dans la solution aqueuse du milieu de culture cellulaire.

10. Procédé selon la revendication 9, dans lequel la cellule est une cellule d'une lignée cellulaire primaire ou d'une lignée cellulaire continue et est de préférence choisie dans le groupe constitué par une cellule de mammifère, une cellule d'oiseau et une cellule d'insecte, dans lequel la cellule est de préférence une cellule de mammifère, de préférence une cellule de mammifère choisie dans le groupe constitué par CHO, NSO, SP2/0, des hybridomes, HEK293, PERC-6, BHK-21 et Vero-76, plus préférablement une cellule CHO, en particulier une cellule CHO choisie dans le groupe constitué par CHO-DG44, CHO-DUKX, CHO-S et CHO-K1.

11. Procédé de fabrication d'au moins une protéine recombinante dans une culture cellulaire, dans lequel le procédé selon l'une des revendications 9 ou 10 comprend en outre l'introduction d'un acide nucléique dans l'au moins une cellule, dans lequel l'acide nucléique provoque une production constitutive ou induite d'au moins une protéine recombinante, provoque de préférence en outre une sécrétion de la protéine produite dans la solution aqueuse du milieu de culture cellulaire, dans lequel la protéine recombinante est tout particulièrement choisie dans le groupe constitué par une protéine thérapeutique, un anticorps, une protéine de fusion, une enzyme, un vaccin, un biosimilaire et les combinaisons de ceux-ci, de préférence choisie dans le groupe constitué par une kinase, une hormone, un anticorps, un vaccin, une protéine de fusion de protéine rapporteuse et les combinaisons de ceux-ci.

12. Utilisation d'un complexe fer-citrate-diphosphate dont le numéro CAS est 85338-24-5 dans un milieu de culture cellulaire, dans laquelle le milieu de culture cellulaire se présente sous forme dissoute et contient un complexe fer-citrate-diphosphate dans une quantité telle que la concentration en fer du milieu de culture cellulaire déterminée par le complexe fer-citrate-diphosphate est comprise entre 80 µM et 5 800 µM.
